# EUROPEAN PATENT APPLICATION

(11) **EP 4 364 705 A1**
(43) Date of publication of application: **08.05.2024**
(21) Application number: 22831575.0
(22) Date of filing: 30.05.2022
(51) Int. Cl.: A61F 2/962, A61F 2/07

(54) **CONVEYING DEVICE AND MEDICAL DEVICE**

(30) Priority: 30.06.2021 CN 202110735964
(71) Applicant: Shanghai Intervascular MedTech Co., Ltd., Shanghai 201318 (CN)
(72) Inventor: CAO, Yang, Shanghai 201318 (CN); ZHANG, Yu, Shanghai 201318 (CN); WANG, Jinyao, Shanghai 201318 (CN); ZHU, Qing, Shanghai 201318 (CN)
(74) Representative: Lavoix
(86) International application number: PCT/CN2022/095872
(87) International publication number: WO 2023/273753

(57) **Abstract**

A conveying device (1000) and a medical device. The conveying device (1000) comprises a catheter mechanism (1100) and a limiting body (1200); wherein the catheter mechanism (1100) comprises an inner tube assembly (1110) and an outer tube (1120), and the outer tube (1120) is sleeved on the inner tube assembly (1110) and is configured to be movable along an axial direction of the inner tube assembly (1110); the limiting body (1200) is connected to a distal outer surface of the inner tube assembly (1110) and is configured to expand or contract along a radial direction of the inner tube assembly (1110); the conveying device (1000) is configured such that the catheter mechanism (1100) is configured to load the medical stent (2000) and to release the medical stent (2000) when the outer tube (1120) moves from distal to proximal, and the limiting body (1200) is configured to expand when the medical stent (2000) is released so as to position the medical stent (2000) in a target position. When the conveying device (1000) releases the medical stent (2000), it can effectively avoid the problem of inaccurate positioning caused by jump forward of the medical stent (2000).

## Description

### TECHNICAL FIELD

The present invention relates to the technical field of medical instruments and, more specifically, to a conveying device and a medical device.

### BACKGROUND

Transjugular intrahepatic portosystemic shunt (TIPS) is a minimally invasive treatment technology that implants a stent between the portal vein and hepatic vein and establishes a portosystemic shunt channel to reduce portal vein pressure. It is used to treat portal hypertension, refractory ascites, esophageal and gastric variceal bleeding caused by portal hypertension and other conditions.

During the process of transjugular intrahepatic portosystemic shunt, transjugular intrahepatic puncture is first performed to establish an artificial channel, and then the covered stent for TIPS is transported along the artificial channel and released at the target location, thereby establishing a portosystemic shunt channel and reducing blood flow pressure in the portal vein.

The covered stent for TIPS usually include a bare segment and a covered segment. The bare segment has a length about 20 mm, and is used to be positioned in the portal vein to ensure that the blood perfusion from the portal vein to other branches is not affected. The covered segment has a length about 40mm-80mm, and is used to be positioned in the liver and hepatic vein to effectively prevent from bile erosion. The introduction path of the covered stent for TIPS is as follows: it first enters the inferior vena cava through the jugular vein approach, then enters the hepatic vein through the inferior vena cava, then exits from the hepatic vein puncture port and passes through the liver parenchyma into the portal vein, and then the bare segment is released in the portal vein, the conveying device and the covered stent for TIPS are then withdrawn as a whole until the junction between the bare segment and the covered segment coincides with the portal vein puncture port, and finally the covered segment is released. When the conveying device in the prior art releases the covered segment, the coated segment is prone to jump forward, causing the covered segment to at least partially enter the portal vein, thereby causing the risk of blocking the portal vein branches.

### SUMMARY OF THE INVENTION

An object of the present invention is to provide a conveying device and a medical device, which can be used to release a medical stent and can effectively prevent the medical stent from jumping forward during release and improve the positioning accuracy of the medical stent when released.

To achieve the above object, the present invention provides a conveying device, comprising a catheter mechanism and a limiting body; wherein the catheter mechanism comprises an inner tube assembly and an outer tube, and the outer tube is sleeved on the inner tube assembly and is configured to be movable along an axial direction of the inner tube assembly; the limiting body is connected to a distal outer surface of the inner tube assembly and is configured to expand or contract along a radial direction of the inner tube assembly;
the conveying device is configured such that the catheter mechanism is configured to load a medical stent and to release the medical stent when the outer tube moves from distal to proximal, and the limiting body is configured to expand when the medical stent is released so as to position the medical stent in a target position.

Optionally, the medical stent comprises a covered segment and a bare segment connected to a distal end of the covered segment;
wherein the conveying device is configured such that the limiting body applies a radial expansion force to the bare segment when the bare segment is released and the limiting body expands so as to expands the bare segment until a current outer surface thereof is larger than a outer surface in a natural state.

Optionally, the limiting body comprises a balloon.

Optionally, the conveying device further comprises a first developing element configured to indicate a position of the limiting body.

Optionally, the inner tube assembly comprises a first tube body and a second tube body sleeved on the first tube body, and a fluid channel is formed between an inner wall of the second tube body and an outer wall of the first tube body; the balloon is sleeved on the first tube body, the balloon has a distal end connected to a distal end of the first tube body, and a proximal end connected to a distal end of the second tube body, and the balloon is in communication with the fluid channel.

Optionally, the inner tube assembly further comprises a third tube body, a fourth tube body and a lubrication tube; both the third tube body and the fourth tube body are sleeved on the second tube body, a proximal end of the fourth tube body is connected to a distal end of the third tube body, and the fourth tube body has a cylindrical spring structure extending along an axis of the inner tube assembly; the lubrication tube is sleeved on the fourth tube body; and/or
the inner tube assembly further comprises an ejection head connected to a distal end of the fourth tube body, a first step surface facing toward a distal end of the inner tube assembly is formed on the ejection head, and the first step surface is configured to abut against a proximal end of the medical stent.

Optionally, the outer tube comprises a lubrication layer, a metal braided layer and a basal layer, which are arranged in sequence from inside to outside.

Optionally, the outer tube further comprises a plurality of fibers evenly arranged along a circumferential direction of the outer tube, and each of the fibers is arranged between the lubrication layer and the metal braided layer and extends along an axial direction of the outer tube.

Optionally, the conveying device further comprises a guide mechanism connected to a distal end of the inner tube assembly; the guide mechanism has a proximal end configured to be disposed inside the outer tube, and a distal outer surface smoothly transiting to an outer surface of the outer tube.

Optionally, the conveying device further comprises a handle assembly comprising a housing, a transmission member and a driving member; the housing is connected to a proximal end of the inner tube assembly and is configured to remain stationary relative to the inner tube assembly; the transmission member is partially disposed inside the housing and is connected to a proximal end of the outer tube; the driving member is disposed on the housing and is configured to drive the transmission member to move along the axial direction of the inner tube assembly so as to drive the outer tube to move along the axial direction of the inner tube assembly.

Optionally, the housing defines thereon an avoidance slot extending along the axial direction of the inner tube assembly; the transmission member comprises a rack partially disposed inside the housing, and the rack comprises teeth extending out of the housing from the avoidance slot; the driving member is sleeved on the housing, and a inner wall of the driving member is provided with internal threads that engage with the rack;
the conveying device is configured such that the driving member and the transmission member form a screw transmission when the driving member rotates around an axis of the inner tube assembly, so that the transmission member is driven to move along the axial direction of the inner tube assembly; the conveying device is further configured such that the transmission member is driven to move along the axial direction of the inner tube assembly when the driving member moves on the housing along the axial direction of the inner tube assembly.

Optionally, the handle assembly further comprises a locking structure provided on the housing and configured to selectively connect with or disconnect from the driving member; when the locking structure is connected to the driving member, the driving member is prevented from driving the transmission member to move along the axial direction of the inner tube assembly; when the locking structure is disconnected from the driving member, the driving member is allowed to drive the transmission member to move along the axial direction of the inner tube assembly.

Optionally, the driving member defines on an inner wall thereof a wavy locking groove extending in a circle along a circumferential direction of the inner tube assembly; the locking structure comprises an operating member and a locking member; the operating member is partially located inside the housing and is rotatably connected to the housing; the operating member has a rotation axis parallel to an axis of the inner tube assembly; the locking member comprises an eccentric boss provided inside the housing and connected to the operating member, and the eccentric boss is configured to rotate with a rotation of the operating member;
the conveying device is configured such that the locking structure is connected to the driving member when the eccentric boss is at least partially inserted into the locking groove and abuts against a wall of the locking groove, and that the locking structure is disconnected from the driving member when the operating member rotates to drive the eccentric boss to separate from the locking groove.

To achieve the above object, the present invention also provides a medical device, comprising the conveying device according to the above medical stent, wherein the medical stent comprises a covered segment and a bare segment connected to a distal end of the covered segment; the medical stent is configured to be sleeved on a distal end of the inner tube assembly of the conveying device and compressed within the outer tube of the conveying device, and the bare segment covers the limiting body of the conveying device.

Optionally, the medical stent comprises a second developing element disposed at a junction between the covered segment and the bare segment.

Compared with the prior art, the conveying device and the medical device according to the present invention have the following advantages:
The aforementioned medical device includes a conveying device and a medical stent, and the conveying device includes a catheter mechanism and a limiting body. The catheter mechanism includes an inner tube assembly and an outer tube. The outer tube is sleeved on the inner tube assembly and is configured to be movable along the axial direction of the inner tube assembly. The limiting body is connected to the distal outer surface of the inner tube assembly, and is configured to be able to expand or contract along the radial direction of the inner tube assembly. The medical stent includes a covered segment and a bare segment. The bare segment is connected to the distal end of the covered segment. The medical stent is configured to be sleeved on the inner tube assembly and compressed in the outer tube. In the use of the medical device for TIPS, after the bare segment is released at the portal vein, the position and posture of the medical stent is adjusted so that the junction between the bare segment and the covered segment coincides with the puncture port of the portal vein, and then the limiting body can be radially expand to provides a radial expansion force to the bare segment, so that the bare segment is further expanded until its current outer diameter is greater than the outer diameter in its natural state. In this way, a greater radial expansion force can be provided from the junction between the bare segment and the covered segment to the puncture port of the portal vein so that the medical stent is stuck and thus fixed at the puncture port of the portal vein. Subsequentially, the covered segment is released. As a result, the covered segment is prevented from jumping forward, thereby improving the positioning accuracy of the medical stent.

### BRIEF DESCRIPTION OF THE DRAWINGS

The present invention will be better understood with reference to the accompanying drawings provided without limiting the invention, in which:
Fig. 1 is a schematic diagram showing an overall structure of a conveying device of a medical device according to an embodiment of the application;
Fig. 2 is a schematic diagram showing a partially enlarged view of a medical device according to an embodiment of the present invention, in which the medical stent is entirely compressed in the catheter mechanism;
Fig. 3 is a schematic diagram showing a partially enlarged view of a medical device according to an embodiment of the present invention, in which the outer tube is moved back to release the bare segment of the medical stent, and the limiting body radially expands;
Fig. 4 is a schematic diagram showing a usage scenario of the medical device according to an embodiment of the present invention, in which the conveying device conveys at least the bare segment of the medical stent to the portal vein;
Fig. 5 is a schematic diagram showing a usage scenario of the medical device according to an embodiment of the present invention, in which the bare segment of the medical device has been released, but the limiting body has not expanded radially;
Fig. 6 is a schematic diagram showing a usage scenario of the medical device according to an embodiment of the present invention, in which the limiting body radially expands;
Fig. 7 is a schematic diagram showing a partially enlarged view of the usage scenario shown in Fig. 6;
Fig. 8 is a schematic diagram showing a usage scenario of the medical device according to an embodiment of the present invention, in which the covered segment of the medical stent has been released;
Fig. 9 is a schematic diagram showing a partial structure of the medical device according to an embodiment of the present invention, in which the third tube body, the fourth tube body, the lubrication tube and the ejection head of the conveying device are shown;
Fig. 10 is a schematic diagram showing a partial structure of the conveying device of the medical device according to an embodiment of the present invention, in which the structure of the ejection head is shown;
Fig. 11 is a schematic diagram showing a partial structure of the conveying device of the medical device according to an embodiment of the present invention, in which the cooperative relationship between the catheter mechanism and the guide mechanism is shown;
Fig. 12 is a cross-sectional view of the guide mechanism of the medical device according to an embodiment of the present invention;
Fig. 13 is a schematic diagram showing a partial structure of the conveying device of the medical device according to an embodiment of the present invention, in which the cooperative relationship between the handle assembly and the catheter mechanism is shown;
Fig. 14 is a schematic diagram showing the cooperative relationship between the driving member and the rack of the transmission member of the conveying device of the medical device according to an embodiment of the present invention;
Fig. 15 is a schematic diagram showing the cooperative relationship between the locking structure and the driving member of the conveying device of the medical device according to an embodiment of the present invention;
Fig. 16 is a schematic diagram showing the cooperative relationship between the locking structure and the driving member of the conveying device of the medical device according to an embodiment of the present invention, which is viewed in a direction different from that of Fig. 15;
Fig. 17 is a schematic diagram showing the cooperative relationship between the outer tube and the transmission member of the conveying device of the medical device according to an embodiment of the present invention;
Fig. 18 is a cross-sectional view of the outer tube of the medical device according to an embodiment of the present invention;
Fig. 19 is a schematic diagram showing a partial structure of a metal braided layer of the outer tube of the medical device according to an embodiment of the present invention.

List of reference numerals:
1000: Conveying Device; 1100: Catheter Mechanism; 1110: Inner Tube Assembly: 1111: First Tube Body; 1112: Second Tube Body; 1113: Third Tube Body; 1114: Fourth Tube Body; 1115: Lubrication Tube; 1116: Ejection Head; 1116a: First Segment; 1116b: Second Segment; 1116c: First Step Surface; 1120: Outer Tube; 1121: Lubrication Layer; 1122: Metal Braided Layer; 1123: Basal Layer; 1124: Fiber; 1001: First Developing Element; 1200: Limiting Body; 1300: Guide Mechanism; 1310: Through Hole; 1311: Proximal Segment; 1312: Distal Segment; 1320: Second Step Surface; 1330: Discharge Groove; 1400: Handle Assembly; 1410: Housing; 1411: Seat; 1420: Transmission Member; 1421: Rack; 1422a: First Transmission and Engagement Member; 1422b: Second Transmission and Engagement Member; 1423: Sealing Ring; 1430: Driving Member; 1431: Locking Groove; 1440: Locking Structure; 1441: Operating Member; 1442: Eccentric Boss; 1450: Stopper; 1500: Connector Assembly; 1510: First Connector; 1520: Second Connector; 1600: Infusion Assembly;
2000: Medical Stent; 2100: Covered Segment; 2200: Bare Segment; 2300: Second Developing Element;
10: Guide Wire.

### DETAILED DESCRIPTION

The following describes the embodiments of the present invention through specific examples. Those skilled in the art can easily understand other advantages and effects of the present invention from the content disclosed in this specification. The present invention can also be implemented or applied through other different specific embodiments. Various details in this specification can also be modified or changed in various ways based on different viewpoints and applications without departing from the spirit of the present invention. It should be noted that the diagrams provided in this embodiment only illustrate the basic concept of the present invention in a schematic manner. The drawings only show the components related to the present invention and do not follow the actual implementation of the components in their numbers, shapes and dimensions. In actual implementation, the type, quantity and proportion of each component can be arbitrarily changed, and the component distribution may also be more complex.

In addition, each embodiment described below has one or more technical features, but this does not mean that the inventor must implement all the technical features in any embodiment at the same time, or can only implement some or all of the technical features in different embodiments separately. In other words, on the premise that implementation is possible, those skilled in the art can selectively implement some or all of the technical features in any embodiment based on the disclosure of the present invention and design specifications or implementation requirements, or selectively implement a combination of some or all of the technical features in multiple embodiments, thereby increasing the flexibility of the implementation of the present invention.

As used in this specification, the singular forms "a", "an" and "the" include plural referents, and the plural form "plurality" includes two or more referents unless the content clearly dictates otherwise. As used in this specification, the term "or" is generally used in its sense including "and/or" unless the content clearly dictates otherwise, and the terms "mounted", "connected," and "coupled" shall be understood in a broad sense, for example, it can be a fixed connection, a detachable connection, or an integral connection. The connection can be mechanical or electrical. It can be a direct connection or an indirect connection through an intermediary. It can be an internal connection between two elements or an interaction between two elements. For those of ordinary skill in the art, the specific meanings of the above terms in the present invention can be understood according to specific circumstances.

As used herein, a "proximal end" and a "distal end" are relative orientations, relative positions and directions of elements or actions relative to each other from the perspective of a surgeon using the medical instruments. Although the "proximal end" and the "distal end" are not restrictive, the "proximal end" generally refers to the end of the medical device that is close to the surgeon during normal operation, while the "distal end" generally refers to the end that first enters the patient.

The core object of the present invention is to provide a conveying device and a medical device including the conveying device. The medical device also includes a medical stent. The conveying device is configured to load the medical stent and convey the medical stent to and release it at the target position in the patient's body. The conveying device includes a catheter mechanism and a limiting body. The catheter mechanism includes an inner tube assembly and an outer tube sleeved on the inner tube assembly, and the outer tube is configured to be movable along the axial direction of the inner tube assembly. The limiting body is connected to the distal outer surface of the inner tube assembly and is configured to expand or contract along the radial direction of the inner tube assembly. The medical stent includes a covered segment and a bare segment, and the bare segment is connected to the distal end of the covered segment. The medical stent of the medical device is configured to be sleeved on the distal end of the inner tube assembly and compressed inside the outer tube, and the bare segment covers the limiting body.

The medical device can be used for transjugular intrahepatic portosystemic shunt, and the medical stent is thus a covered stent for TIPS. The conveying device is configured to convey the medical stent into the human body and release the bare segment in the portal vein, and release the covered segment within the liver parenchyma. During actual use, when the bare segment is released and the position and posture of the medical stent is adjusted so that the junction between the bare segment and the covered segment coincides with the puncture port on the portal vein (i.e., the position of the medical stent is the aforementioned target position), the operator can radially expand the limiting body through relevant operations, and then the limiting body provides a radial expansion force to the bare segment, so that the bare segment is further expanded until its current outer diameter is greater than the outer diameter in its natural state. In this way, the medical stent provides a greater radial expansion force to the puncture port of the portal vein so as to fix the medical stent. Subsequentially, the covered segment is released. As a result, the covered segment is prevented from jumping forward, thereby improving the positioning accuracy in the release of the medical stent. Those skilled in the art will know that "the outer diameter in its natural state" refers to the outer diameter of the bare segment when the medical stent is not subjected to a radial force (including a radial extrusion force and a radial expansion force).

To make the objectives, advantages and features of the present invention clearer, the present invention will be further described in detail below with reference to the figures. It is noted that the figures are provided in a very simplified form not necessarily presented to scale, with their only intention to facilitate convenience and clarity in explaining the disclosed embodiments. Throughout the figures, like reference numerals indicate the same or analogous components or elements.

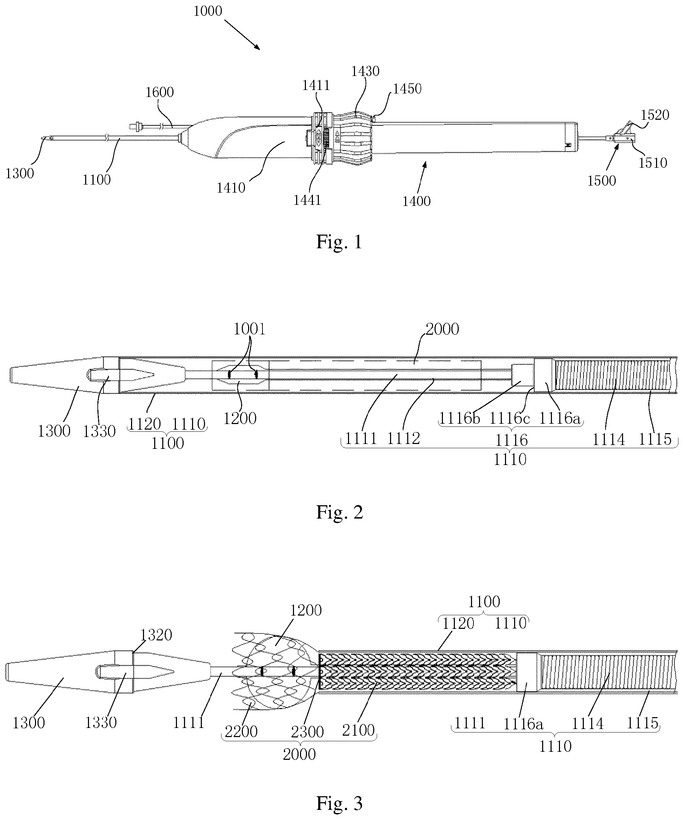

Fig. 1 is a schematic diagram showing an overall structure of a conveying device of a medical device according to an embodiment of the application; Fig. 2 is a schematic diagram showing a partially enlarged view of a medical device in an state; Fig. 3 is a schematic diagram showing a partially enlarged view of a medical device in another state.

Please refer to Figs. 1 to 3, the medical device includes a conveying device 1000 and a medical stent 2000. The conveying device 1000 includes a catheter mechanism 1100 and a limiting body 1200. The catheter mechanism 1100 includes an inner tube assembly 1110 and an outer tube 1120. The outer tube 1120 is sleeved on the inner tube assembly 1110 and is configured to be movable along the axial direction of the inner tube assembly 1110. The limiting body 1200 is connected to the distal outer surface of the inner tube assembly 1110 and is configured to expand or contract along the radial direction of the inner tube assembly 1110. The medical stent 2000 includes a covered segment 2100 and a bare segment 2200. The bare segment 2200 is connected to the distal end of the covered segment 2100. The medical stent 2000 is used to be sleeved on the distal end of the inner tube assembly 1110 and compressed into the outer tube 1120, and the bare segment 2200 covers the limiting body 1200.

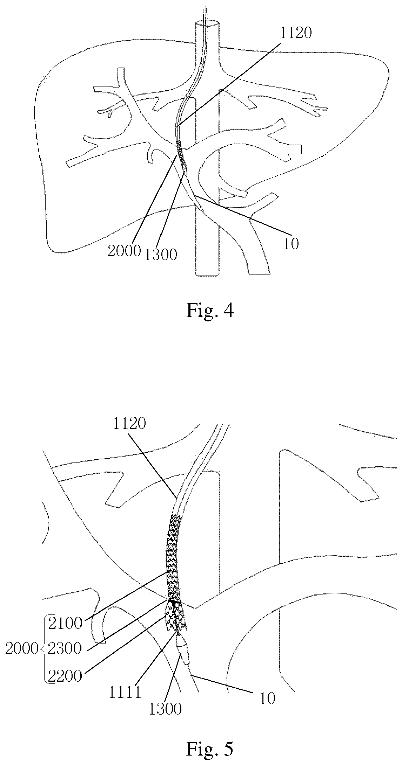

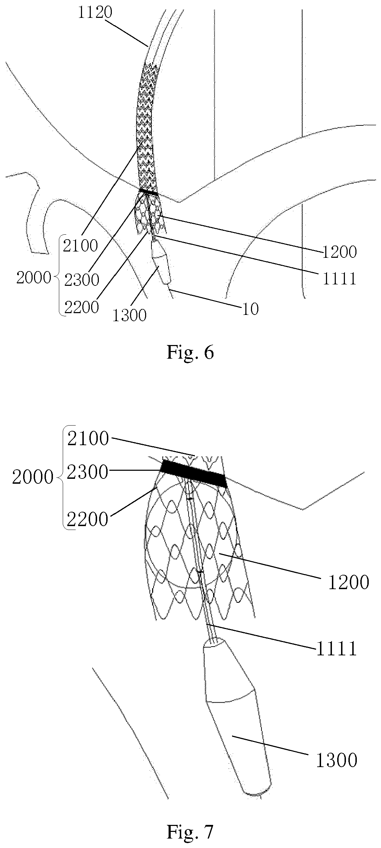

When transjugular intrahepatic portosystemic shunt is performed, as shown in Figs. 4 to 8, the conveying device 1000 first conveys the medical stent 2000 into the human body so that at least the bare segment 2200 completely accesses to the portal vein after passing through the jugular vein, inferior vena cava, right hepatic vein, and liver parenchyma. The operator then controls the outer tube 1120 to move from distal to proximal to release the bare segment 2200. After that, the position and posture of the medical stent 2000 is adjusted so that the junction between the bare segment 2200 and the covered segment 2100 coincides with the puncture port of the portal vein. The operator then performs relevant operations to radially expand the limiting body 1200 so that the limiting body 1200 applies a radial expansion force to the bare segment 2200 to cause the bare segment 2200 to further expand until is current outer diameter is greater than the outer diameter in its natural state. As a result, the junction between the bare segment 2200 and the covered segment 2100 applies a great radial support force to the portal vein puncture port so that the medical stent 2000 is fixed under the pressure of the portal vein puncture port. After that, the operator controls the outer tube 1120 to move from distal to proximal to release the covered segment 2100. Since the medical stent 2000 has been fixed, the covered segment 2100 can be prevented from jumping due to the sudden change of radial force when released. Therefore, the shift of the medical stent 2000 is avoided, and the positioning accuracy of the medical stent 2000 is improved.

Optionally, the conveying device 1000 further includes a first developing element 1001 configured to indicate the position of the limiting body 1200. The medical stent 2000 further includes a second developing element 2300 configured to indicate the position of the junction between the covered segment 2100 and the bare segment 2200. The second developing element 2300 is preferably provided at the junction between the covered segment 2100 and the bare segment 2200.

In a non-limiting embodiment, the limiting body 1200 includes a balloon. Correspondingly, please mainly refer to Fig. 2, the inner tube assembly 1110 includes a first tube body 1111 and a second tube body 1112. The second tube body 1112 is sleeved on the first tube body 1111, and a fluid channel (not labeled in the figure) is formed between the inner wall of the second tube body 1112 and the outer wall of the first tube body 1111. The balloon is sleeved on the first tube body 1111, the distal end of the balloon is connected to the distal end of the first tube body 1111, and the proximal end of the balloon is connected to the distal end of the second tube body 1112, and the balloon is also in communication with the fluid channel. That is to say, the operator can inject the filling agent into the balloon through the fluid channel to inflate the balloon (that is, to radially expand the balloon), and discharge the filling agent from the balloon to deflate the balloon (that is, to radially contract the balloon). The filling agent is, for example, contrast agent or physiological saline. In this embodiment, the number of the first developing elements 1001 may be two, and the two first developing elements 1001 are disposed on the outer surface of the first tube body 1111 and arranged in correspondence to the proximal and distal ends of the balloons respectively.

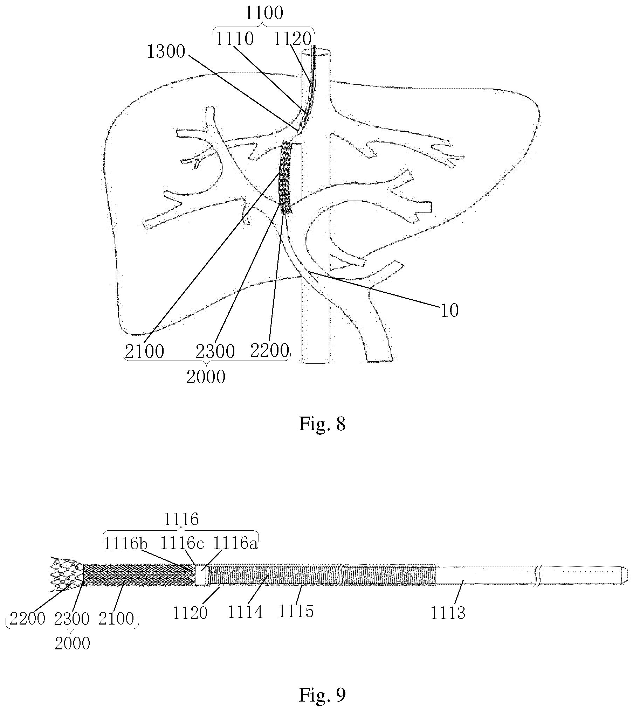

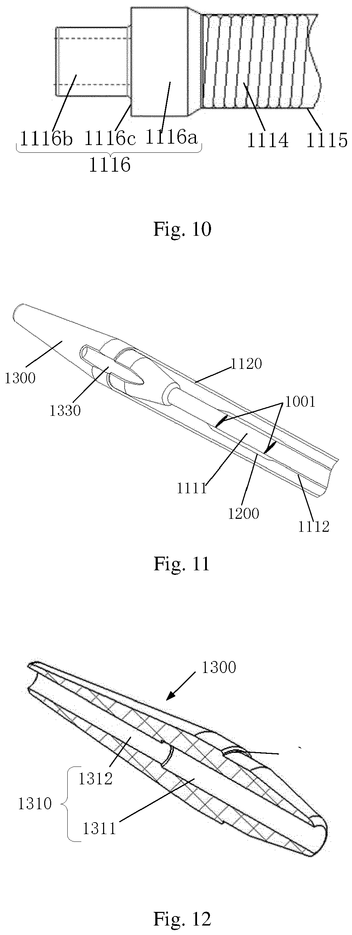

Please refer to Figs. 9 and 10, the inner tube assembly 1110 further includes a third tube body 1113, a fourth tube body 1114 and a lubrication tube 1115. The third tube body 1113 and the fourth tube body 1114 are both sleeved on the second tube body 1112, and the proximal end of the fourth tube body 1114 is connected to the distal end of the third tube body 1113. In this embodiment, the fourth tube body 1114 has a cylindrical spring structure formed by spirally winding a wire around the axis of the inner tube assembly 1110. Such a structure is conducive to improving the flexibility of the inner tube assembly 1110 so that it is easier to pass through tortuous blood vessels. The lubrication tube 1115 is sleeved on the fourth tube body 1114. The lubrication tube 1115 is, for example, a PTFE tube, which is fixed on the outer surface of the fourth tube body 1114 through heat shrink process to reduce the friction force generated from the axial movement of the outer tube 1120 relative to the inner tube assembly 1110.

Further, the inner tube assembly 1110 further includes an ejection head 1116 connected to the distal end of the fourth tube body 1114. The ejection head 1116 includes a first segment 1116a and a second segment 1116b which is closer to the distal end of the inner tube assembly 1110. The outer diameter of the second segment 1116b is smaller than the outer diameter of the first segment 1116a. In this way, the first step surface 1116c facing toward the distal end of the inner tube assembly 1110 is formed on the ejection head 1116. The first step surface 1116c is configured to abut against the proximal end of the medical stent 2000, so that the medical stent 2000 can be positionally limited by the first step surface 1116c when the outer tube 1120 moves from distal to proximal. As a result, the medical stent 2000 is prevented from moving proximally along with the outer tube 1120 under the action of the friction force, thereby ensuring smooth release of the medical stent 2000.

Further, please refer back to Figs. 2 and 3, and in conjunction with Fig. 11, the conveying device further includes a guide mechanism 1300. The guide mechanism 1300 is connected to the distal end of the inner tube assembly 1110, and configured to guide the conveying device 1000.

In this embodiment, the outer diameter of the guide mechanism 1300 first increases and then decreases from proximal to distal. As shown in Fig. 12, the guide mechanism 1300 has an axial through hole 1310. The through hole 1310 includes a proximal segment 1311 and a distal segment 1312 (labeled in Fig. 12) which are in communication with each other. The proximal segment 1311 is connected to the distal end of the first tube body 1111 of the inner tube assembly 1110, so that the inner cavity of the first tube body 1111 communicates with the distal segment 1312 and is configured for passing the guide wire 10 (labeled in Figs. 4 to 8). As a result, the guide mechanism 1300 can travel along the guide wire 10 in the human body and guide the conveying device 1000 to a predetermined position in the human body.

A second step surface 1320 facing toward the proximal end of the inner tube assembly 1110 is formed on the outer wall of the guide mechanism 1300. The second step surface 1320 is configured to abut against the distal end surface of the outer tube 1120. The situation in which the distal end surface of the outer tube 1120 abuts against the second step surface 1320 is herein referred to as the catheter mechanism 1100 being in a closed state. It can be understood that when the catheter mechanism 1100 is in the closed state, the portion of the guide mechanism 1300 proximal to the second step surface 1320 is located inside the outer tube 1120. Furthermore, in a preferred embodiment, when the catheter mechanism 1100 is in the closed state, the portion of the outer surface of the guide mechanism 1300 which is distal to the second step surface 1320 smoothly transits to the outer surface of the outer tube 1120. This is advanced in that the conveying device 1000 will not scratch the patient's tissue during its movement in the human body.

In addition, please continue to refer to Fig. 11, the guide mechanism 1300 further defines on its outer surface a discharge groove 1330. The discharge groove 1330 is in communication with the inner cavity of the outer tube 1120 and is configured to discharge the gas or liquid from the outer tube 1120.

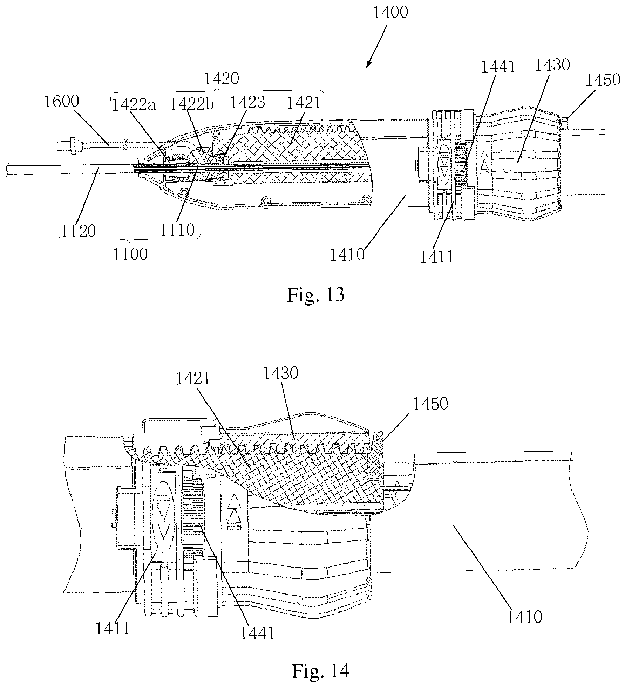

Please refer back to Fig. 1 and in conjunction with Fig. 13, the conveying device 1000 also includes a handle assembly 1400. The handle assembly 1400 includes a housing 1410, a transmission member 1420 and a driving member 1430. The housing 1410 is connected to the proximal end of the inner tube assembly 1110 (specifically, connected to the proximal end of the third tube body 1113) and is configured to remain stationary relative to the inner tube assembly 1110. The transmission member 1420 is partially disposed inside the housing 1410 and connected to the proximal end of the outer tube 1120. The driving member 1430 is disposed on the housing 1410 and is configured to drive the transmission member 1420 to move along the axial direction of the inner tube assembly 1110, and thereby drive the outer tube 1120 to move along the axial direction of the inner tube assembly 1110.

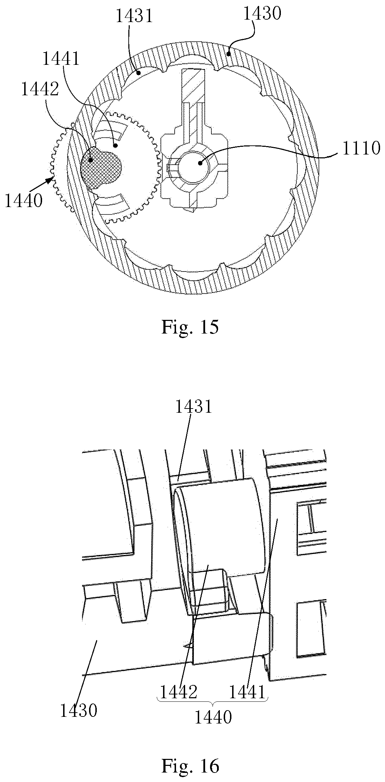

In practice, the operator hopes to release the bare segment 2200 of the medical stent 2000 smoothly and stably, and to release the covered segment 2100 quickly, so as to improve the safety of the operation and shorten the operation time. In view of this, please continue to refer to Fig. 13 in conjunction with Figs. 14 and 15, the housing 1410 defines thereon an avoidance slot (not shown in the figures) extending along the axial direction of the inner tube assembly 1110. The transmission member 1420 includes a rack 1421 partially disposed inside the housing 1410. The teeth of the rack 1421 extend out of the housing 1410 from the avoidance slot. The driving member 1430 is sleeved on the housing 1410, and the inner wall of the driving member 1430 is provided with internal threads that engage with the rack 1421. The conveying device is configured such that the driving member 1430 and the rack 1421 form a screw transmission when the driving member 1430 rotates around the axis of the inner tube assembly 1110, so that the transmission member 1420 moves along the axial direction of the inner tube assembly 1110. When the driving member 1430 moves along the axial direction of the inner tube assembly 1110, the transmission member 1420 is driven to move along the axial direction of the inner tube assembly 1110.

That is to say, the driving member 1430 can either drive the transmission member 1420 to move along the axial direction of the inner tube assembly 1110 through screw transmission, or drive the transmission member along the axial direction of the inner tube assembly 1110 through direct pulling. Those skilled in the art know that the screw transmission is characterized in that it is stable and the stroke can be controlled more accurately, while the direct pulling allows a rapid movement. Therefore, when releasing the medical stent 2000, the operator can first release the bare segment 2200 through screw transmission, and then release the covered segment 2100 through direct pulling.

It should be noted that the proximal end of the inner tube assembly 1110 may extend out of the housing 1410 and be connected to a connector assembly 1500 (as shown in Fig. 1). The connector assembly 1500 includes a first connector 1510 and a second connector 1520. The first connector 1510 is in communication with the inner cavity of the first tube body 1111 of the inner tube assembly 1110 and is used for passing the guide wire 10. The second connector 1520 is in communication with the fluid channel of the inner tube assembly 1110 and is used for filling or discharging the filling agent.

It should also be noted that in this embodiment, as shown in Fig. 15, the transmission member 1420 further includes two transmission and engagement members, which are a first transmission and engagement member 1422a and a second transmission and engagement member 1422b. The first transmission and engagement member 1422a is injection molded on the proximal end of the outer tube 1120. The second transmission and engagement member 1422b is sleeved on the inner tube assembly 1110. The distal end of the second transmission and engagement member 1422b is threadedly connected to the first transmission and engagement member 1422a. The proximal end of the second transmission and engagement member 1422b is connected to the distal end of the rack 1421. In addition, a sealing ring 1423 is provided between the proximal end of the second transmission and engagement member 1422b and the inner tube assembly 1110. In addition, similar to the prior art, the conveying device 1000 according to the embodiment of the present invention also includes an infusion assembly 1600. One end of the infusion assembly 1600 extends into the interior of the housing 1410 and is connected to the second transmission and engagement member 1422b, and is in communication with the outer tube 1120 so as to allow liquid to be infused into the patient's body through the inner cavity of the outer tube 1120, or to allow liquid or gas to be discharged from the patient's body through the inner cavity of the outer tube 1120.

Preferably, please mainly refer to Figs. 13 to 16, the handle assembly 1400 further includes a locking structure 1440, which is provided on the housing 1410 and configured to selectively engage with or disengage from the driving member 1430. When the locking structure 1440 is connected to the driving member 1430, the driving member 1430 is prevented from driving the transmission member 1420 to move along the axial direction of the inner tube assembly 1110. When the locking structure disengages from the driving member 1430, the driving member 1430 is allowed to drive the transmission member 1420 to move along the axial direction of the inner tube assembly 1110.

In this embodiment, when the locking structure 1440 is connected to the driving member 1430, it actually prevents the driving member 1430 from moving on the housing 1410 (i.e., from rotating around the axis of the inner tube assembly 1110 or moving along the axis of the inner tube assembly 1110), thereby preventing the driving member 1430 from driving the transmission member 1420 to move.

Specifically, please refer to Figs. 13 and 14, the driving member 1430 defines on its inner wall a wavy locking groove 1431 extending in a circle along the circumferential direction of the inner tube assembly 1110. The locking groove 1431 is preferably defined at the distal end of the driving member 1430. The locking structure 1440 includes an operating member 1441 and a locking member 1442. A part of the operating member 1441 is located inside the housing 1410 and the other part is located outside the housing 1410 to facilitate the operator's operation. The operating member 1441 is rotatably connected to the housing 1410. In practice, the housing 1410 is provided with a seat 1411, and the operating member 1441 is rotatably connected to the seat 1411. The rotation axis of the operating member 1441 is parallel to the axis of the inner tube assembly 1110. The locking member 1442 includes an eccentric boss 1442, which is connected to the operating member 1441 and configured to rotate with the rotation of the operating member 1441. When the eccentric boss 1442 is at least partially inserted into the locking groove 1431 and abuts against the wall of the locking groove 1431, the locking structure 1440 is connected to the driving member 1430. When the operating member 1441 rotates and then drives the eccentric boss 1442 to rotate until the eccentric boss 1442 is separated from the locking groove 1431, the locking structure 1440 is disconnected from the driving member 1430. The unlocking direction can also be marked on the seat 1411. When the operator rotates the operating member 1441 in the unlocking direction, the connection between the locking structure 1440 and the driving member 1430 can be smoothly released.

In this way, during the process of the conveying device 1000 conveying the medical stent 2000 into the human body, the locking structure 1440 remains connected to the driving member 1430. Before starting to release the medical stent 2000, the operator rotates the operating member 1441 and releases the connection between the locking structure 1440 and the driving member 1430. Subsequently, the operator rotates the driving member 1430 in the first direction to slowly and steadily release the bare segment 2200 of the medical stent 2000. After adjusting the position and posture of the medical stent 2000, the operator injects filling agent into the balloon to expand the balloon. Afterwards, the operator pulls the driving member 1430 proximally to quickly release the covered segment 2100 of the medical stent 2000. After the release of the covered segment 2100 is completed, the operator discharges the filling agent from the balloon to cause the balloon to shrink. After that, the operator also controls the driving member 1430 to move the outer tube 1120 from proximal to distal, and rotates the driving member 1430 in the second direction until the catheter mechanism 1100 is closed, and finally the conveying device 1000 is withdrawn from the patient's body. Here, the second direction is opposite to the first direction.

Optionally, the handle assembly 1400 further includes a stopper 1450, which is configured to prevent the driving member 1430 from being separated from the transmission member 1420. The number of the stopper 1450 may be one, and the stopper 1450 is provided at the proximal end of the rack 1421 in order to prevent the driving member 1430 from being separated at the proximal end of the rack 1421 from the transmission member 1420 when the operator rotates the driving member 1430 in the second direction.

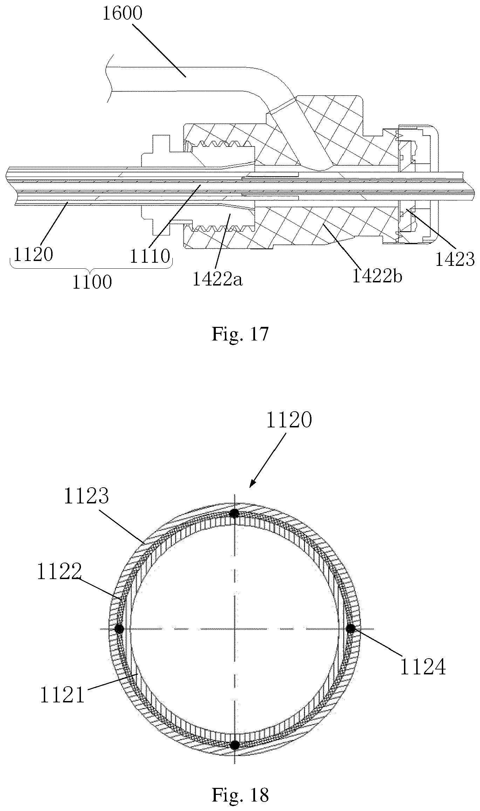

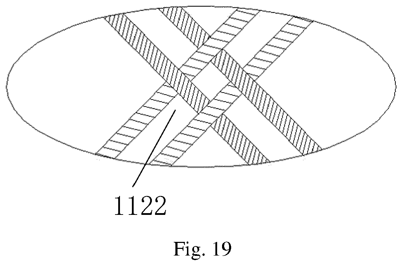

In addition, please refer to Figs. 18 and 19, in the embodiment of the present invention, the outer tube 1120 includes a lubrication layer 1121, a metal braided layer 1122 and a basal layer 1123, which are arranged in sequence from the inside to the outside. The material of the lubrication layer 1121 may be PTFE, which has a small friction coefficient and is beneficial to reducing the frictional force between the medical stent 2000 and the inner tube assembly 1110, which is generated from the movement of the outer tube 1120 along the axial direction of the inner tube assembly 1110. This reduces the resistance when the medical stent 2000 is released. The metal braided layer 1122 can be formed by braiding at least one metal wire. In the embodiment of the present invention, the way of braiding for forming the metal braided layer 1122 is not particularly limited. For example, one metal wire is alternated with another metal wire in sequence. The arrangement of the metal braided layer 1122 can effectively reduce the outer diameter of the outer tube 1120 and reduce the outer diameter of the conveying device while ensuring the torsion control and support of the outer tube 1120. When the conveying device is introduced into the patient's body, it can be used with a smaller sheath to reduce harm to the patient. The basal layer 1123 may be made of Pebax.

Further, the outer tube 1120 further includes a plurality of fibers 1124. The number of the fibers 1124 is usually four, and the four fibers 1124 are evenly arranged between the metal braided layer 1122 and the lubrication layer 1121 along the circumferential direction of the outer tube 1120. Each fiber 1124 extends along the axial direction of the outer tube 1120. The fiber 1124 may be made of aromatic liquid crystal polyester, which is used to strengthen the axial strength of the outer tube 1120.

Based on the above, in the technical solution provided by the present invention, after the bare segment of the medical stent is released and the adjustment of the position and posture of the medical stent is completed, the limiting body is used to provide radial support force to the bare segment, so that the bare segment further radially expands, thereby increasing the radial support force exerted on the portal vein puncture port from the junction between the bare segment and the covered segment, and under the action of this radial support force, the medical stent is maintained at the target position and is prevented from jumping forward when the covered segment is released. Therefore, the displacement of the medical stent is avoided.

Although the present invention is disclosed above, it is not limited thereto. It is apparent that those skilled in the art can make various modifications and variations to the present invention without departing from the spirit and scope thereof. Accordingly, the invention is intended to embrace all such modifications and variations if they fall within the scope of the appended claims and equivalents thereof.

## Claims

1. A conveying device, comprising a catheter mechanism and a limiting body; wherein the catheter mechanism comprises an inner tube assembly and an outer tube, and the outer tube is sleeved on the inner tube assembly and is configured to be movable along an axial direction of the inner tube assembly; the limiting body is connected to a distal outer surface of the inner tube assembly and is configured to expand or contract along a radial direction of the inner tube assembly;
the conveying device is configured such that the catheter mechanism is configured to load a medical stent and to release the medical stent when the outer tube moves from distal to proximal, and the limiting body is configured to expand when the medical stent is released so as to position the medical stent in a target position.

2. The conveying device according to claim 1, wherein the medical stent comprises a covered segment and a bare segment connected to a distal end of the covered segment;
wherein the conveying device is configured such that the limiting body applies a radial expansion force to the bare segment when the bare segment is released and the limiting body expands so as to expands the bare segment until a current outer surface thereof is larger than a outer surface in a natural state.

3. The conveying device according to claim 1 or 2, wherein the limiting body comprises a balloon.

4. The conveying device according to claim 1 or 2, wherein the conveying device further comprises a first developing element configured to indicate a position of the limiting body.

5. The conveying device according to claim 3, wherein the inner tube assembly comprises a first tube body and a second tube body sleeved on the first tube body, and a fluid channel is formed between an inner wall of the second tube body and an outer wall of the first tube body; the balloon is sleeved on the first tube body, the balloon has a distal end connected to a distal end of the first tube body, and a proximal end connected to a distal end of the second tube body, and the balloon is in communicatino with the fluid channel.

6. The conveying device according to claim 5, wherein the inner tube assembly further comprises a third tube body, a fourth tube body and a lubrication tube; both the third tube body and the fourth tube body are sleeved on the second tube body, a proximal end of the fourth tube body is connected to a distal end of the third tube body, and the fourth tube body has a cylindrical spring structure extending along an axis of the inner tube assembly; the lubrication tube is sleeved on the fourth tube body; and/or
the inner tube assembly further comprises an ejection head connected to a distal end of the fourth tube body, a first step surface facing toward a distal end of the inner tube assembly is formed on the ejection head, and the first step surface is configured to abut against a proximal end of the medical stent.

7. The conveying device according to claim 1, wherein the outer tube comprises a lubrication layer, a metal braided layer and a basal layer, which are arranged in sequence from inside to outside.

8. The conveying device according to claim 7, wherein the outer tube further comprises a plurality of fibers evenly arranged along a circumferential direction of the outer tube, and each of the fibers is arranged between the lubrication layer and the metal braided layer and extends along an axial direction of the outer tube.

9. The conveying device according to claim 1, further comprising a guide mechanism connected to a distal end of the inner tube assembly; the guide mechanism has a proximal end configured to be disposed inside the outer tube, and a distal outer surface smoothly transiting to an outer surface of the outer tube.

10. The conveying device according to claim 1, further comprising a handle assembly comprising a housing, a transmission member and a driving member; the housing is connected to a proximal end of the inner tube assembly and is configured to remain stationary relative to the inner tube assembly; the transmission member is partially disposed inside the housing and is connected to a proximal end of the outer tube; the driving member is disposed on the housing and is configured to drive the transmission member to move along the axial direction of the inner tube assembly so as to drive the outer tube to move along the axial direction of the inner tube assembly.

11. The conveying device according to claim 10, wherein the housing defines thereon an avoidance slot extending along the axial direction of the inner tube assembly; the transmission member comprises a rack partially disposed inside the housing, and the rack comprises teeth extending out of the housing from the avoidance slot; the driving member is sleeved on the housing, and a inner wall of the driving member is provided with internal threads that engage with the rack;
the conveying device is configured such that the driving member and the transmission member form a screw transmission when the driving member rotates around an axis of the inner tube assembly, so that the transmission member is driven to move along the axial direction of the inner tube assembly; the conveying device is further configured such that the transmission member is driven to move along the axial direction of the inner tube assembly when the driving member moves on the housing along the axial direction of the inner tube assembly.

12. The conveying device according to claim 10, wherein the handle assembly further comprises a locking structure provided on the housing and configured to selectively connect with or disconnect from the driving member; when the locking structure is connected to the driving member, the driving member is prevented from driving the transmission member to move along the axial direction of the inner tube assembly; when the locking structure is disconnected from the driving member, the driving member is allowed to drive the transmission member to move along the axial direction of the inner tube assembly.

13. The conveying device according to claim 12, wherein the driving member defines on an inner wall thereof a wavy locking groove extending in a circle along a circumferential direction of the inner tube assembly; the locking structure comprises an operating member and a locking member; the operating member is partially located inside the housing and is rotatably connected to the housing; the operating member has a rotation axis parallel to an axis of the inner tube assembly; the locking member comprises an eccentric boss provided inside the housing and connected to the operating member, and the eccentric boss is configured to rotate with a rotation of the operating member;
the conveying device is configured such that the locking structure is connected to the driving member when the eccentric boss is at least partially inserted into the locking groove and abuts against a wall of the locking groove, and that the locking structure is disconnected from the driving member when the operating member rotates to drive the eccentric boss to separate from the locking groove.

14. A medical device, comprising the conveying device according to any one of claims 1 to 13 and a medical stent, wherein the medical stent comprises a covered segment and a bare segment connected to a distal end of the covered segment; the medical stent is configured to be sleeved on a distal end of the inner tube assembly of the conveying device and compressed within the outer tube of the conveying device, and the bare segment covers the limiting body of the conveying device.

15. The medical device according to claim 14, wherein the medical stent comprises a second developing element disposed at a junction between the covered segment and the bare segment.
